(19) Europäisches Patentamt
European Patent Office
Office européen des brevets

(11) EP 3 483 246 A1

(12) EUROPEAN PATENT APPLICATION

(43) Date of publication:
**15.05.2019 Bulletin 2019/20**

(51) Int Cl.:
*C11D 3/38* (2006.01) *C07K 14/37* (2006.01)
*C11D 1/94* (2006.01) *C11D 11/00* (2006.01)

(21) Application number: **18178698.9**

(22) Date of filing: **20.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.11.2017 EP 17201334**

(71) Applicant: **The Procter & Gamble Company**
**Cincinnati, OH 45202 (US)**

(72) Inventors:
- **BETTIOL, Jean-Luc Philippe**
**1853 Strombeek-Bever (BE)**
- **GONZALES, Denis Alfred**
**1853 Strombeek-Bever (BE)**
- **VELASQUEZ, Juan Esteban**
**Cincinnati, Ohio 45202 (US)**
- **GEARY, Nicholas William**
**Cincinnati, Ohio 45202 (US)**

(74) Representative: **Siddiquee, Sanaul Kabir**
**N.V. Procter & Gamble**
**Services Company S.A.**
**Temselaan 100**
**1853 Strombeek-Bever (BE)**

# (54) CLEANING COMPOSITION COMPRISING HYDROPHOBINS

(57) The present invention is directed to a cleaning composition comprising one or more subclass EAS hydrophobins and a surfactant system comprising one or more anionic surfactants and one or more co-surfactants selected from the group consisting of amphoteric surfactant, zwitterionic surfactant, and mixtures thereof for improve suds longevity. Methods of making and using such cleaning compositions are also provided.

Figure 1

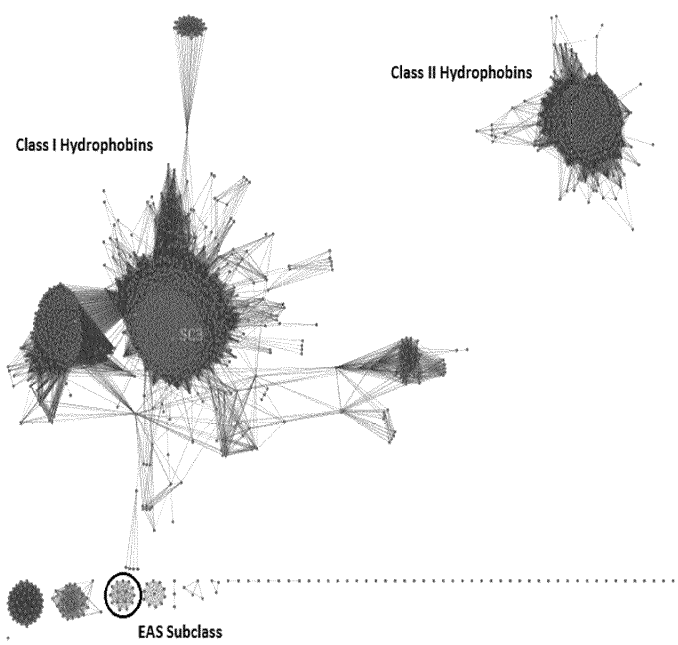


EP 3 483 246 A1

Printed by Jouve, 75001 PARIS (FR)

## Description

REFERENCE TO A SEQUENCE LISTING

This application contains Sequence Listings in computer readable form. The computer readable form is incorporated herein by reference.

FIELD OF THE INVENTION

The present invention relates to a cleaning composition comprising a specific surfactant system and one or more subclass EAS hydrophobins. The composition provides one or more benefits, including good cleaning, long lasting suds especially in presence of greasy soils and surface modification that can contribute to second time cleaning benefits, improved drying, improved shine in the case of dishware.

BACKGROUND OF THE INVENTION

Cleaning compositions should have a good suds profile in particular a long lasting suds profile while providing good soil cleaning. Users usually see suds as an indicator of the performance of the cleaning composition. Moreover, the user of a cleaning composition may also use the suds profile and the appearance of the suds (e.g. density, whiteness) as an indicator that the wash solution still contains active cleaning ingredients. This is particularly the case for manual washing, also referred to herein as hand-washing, where the user usually doses the cleaning composition depending on the suds remaining and renews the wash solution when the suds subside or when the suds does not look thick enough. Thus, a cleaning composition, particularly a manual wash cleaning composition that generates little or low density suds would tend to be replaced by the user more frequently than is necessary. Accordingly, it is desirable for a cleaning composition to provide "good sudsing profile", which includes good suds height and/or density as well as good suds duration during the initial mixing of the composition with water and during the entire washing operation.

Several families of natural surface active proteins are able to produce suds in aqueous solutions (*see* Cooper, A., et al. (2017), Colloids Surf., A: Physiochemical and Engineering Aspects*;* Schor, M., et al. (2016), Trends Biochem. Sci. 41(7): 610-620). Hydrophobins are a class of surface active proteins. Furthermore, detergent compositions containing hydrophobins are known in the art. For example, US 2009/0101167 describes the use of hydrophobins, particularly fusion hydrophobins, for washing textiles and washing compositions containing them. US 2014/0031272 describes a cleaning composition comprising a hydrophobin and a lipolytic enzyme for removing lipid-based stains from surfaces. However, the amount of sudsing generated by such surface active proteins in cleaning formulations is limited.

Accordingly, the need remains for an improved cleaning composition comprising surface active proteins which has a further improved sudsing profile, particularly at low proteins concentrations in the cleaning compositions. The need also exists for an improved cleaning composition, when used in a manual-washing process, the composition preferably also provides a pleasant washing experience, *i.e,* good feel on the user's hands during the wash. Preferably the cleaning compositions are also easy to rinse. Preferably in addition, the composition provides a good finish to the washed items. There is also the desire to reduce the amount of surfactants without negatively impacting sudsing nor grease cleaning and emulsification profile. Thus, there is the need to find new compositions that improve suds longevity in hand washing conditions. The Applicant discovered that some or all of the above-mentioned needs can be at least partially fulfilled through the improved cleaning composition as described herein below.

SUMMARY OF THE INVENTION

The present invention meets one or more of these needs based on the surprising discovery that by formulating a cleaning composition comprising a specific surfactant system and one or more subclass EAS hydrophobins, such a composition exhibits good sudsing profile, particularly desirable suds volume and/or sustained suds stabilization, especially in the presence of greasy soils. It also provides good emulsification benefits and can also provide surface modifications facilitating next time cleaning benefit.

According to a first aspect, the present invention is directed to a cleaning composition comprising from 1 wt% to 60 wt%, preferably from 5 wt% to 50 wt% by weight of said composition of a surfactant system and from 0.001 wt% to 5 wt%, preferably from 0.1 wt% to 1 wt%, by weight of said composition, based on active protein, of one or more subclass EAS hydrophobins according to claim 1. The specific surfactant system comprises one or more anionic surfactants and one or more co-surfactants selected from the group consisting of amphoteric surfactant, zwitterionic surfactant, and mixtures thereof.

Preferably the cleaning composition is a manual-washing cleaning composition. Preferably the cleaning composition is for manual dishwashing. Preferred compositions are in the form of a liquid.

In another aspect, the present invention is directed to a method comprising contacting a cleaning composition of the invention with a surface.

In another aspect, the present invention is directed to a method of manually washing dishware comprising the steps of delivering a detergent composition of the invention into a volume of water to form a wash solution and immersing the dishware in the solution.

In yet another aspect, the present invention is directed to a method of manually washing soiled items comprising contacting a cleaning composition of the invention, wherein said composition modifies the hydrophobicity of said surface as a result of said contacting step.

In yet another aspect, the present invention is directed to a method of improving suds longevity in a washing process for washing soiled articles, preferably dishware. The method comprises the steps of: a) delivering a cleaning composition of the invention to a volume of water to form a wash liquor; and b) immersing the soiled articles into said wash liquor.

In yet another aspect, the present invention relates to a method of manually washing dishware comprising: i) delivering a composition as described herein above onto the dishware or a cleaning implement; ii) cleaning the dishware with the composition in the presence of water; and iii) optionally, rinsing the dishware. Preferably, the composition of the present invention is used in neat form *(i.e.,* direct application) when the composition is directly applied on the soiled surface or on a cleaning implement, such as a sponge, to be used to clean the soiled surface.

In yet another aspect, the present invention is directed to use of one or more subclass EAS hydrophobins of the invention to improve suds longevity in an aqueous wash liquor during a washing process.

It is an object of the composition of the present invention to exhibit good sudsing profile, preferably high suds volume and sustained suds aesthetics *(i.e.,* whiteness, consistency).

It is an object of the composition of the present invention to exhibit good sudsing profile, preferably stable suds during a substantial portion of or for the entire manual dishwashing process.

The elements of the composition of the invention described in relation to the first aspect of the invention apply *mutatis mutandis* to the other aspects of the invention.

These and other features, aspects and advantages of the present invention will become evident to those skilled in the art from the detailed description which follows.

## BRIEF DESCRIPTION OF THE DRAWINGS

While the specification concludes with claims particularly pointing out and distinctly claiming the invention, it is believed that the invention will be better understood from the following description of the accompanying figures:

Figure 1 is a sequence similarity network of hydrophobins identifying the two major classes (class I and class II) and several subclasses including subclass EAS. The network was generated using EFI - Enzyme Similarity Tool Ver 2.0 (*http://efi.igb.illinois.edu/efi-est/*).

## DETAILED DESCRIPTION OF THE INVENTION

### Definitions

As used herein, the articles "a" and "an" when used in a claim, are understood to mean one or more of what is claimed or described.

As used herein, the term "amino acid identity" means the identity between two or more amino acid sequences and is expressed in terms of the identity or similarity between the sequences. Sequence identity can be measured in terms of percentage identity; the higher the percentage, the more identical the sequences are. The percentage identity is calculated over the length of comparison. For example, the amino acid identity is typically calculated over the entire length of a sequence aligned against the entire length of the reference sequence (e.g., SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9, or SEQ ID NO: 11). Methods of alignment of sequences for comparison are well known in the art and identity can be calculated by many known methods. Various programs and alignment algorithms are described in the art. It should be noted that the terms 'sequence identity' and 'sequence similarity' can be used interchangeably.

As used herein, the term "cleaning composition" refers to a composition or formulation designed for cleaning soiled surfaces. Such compositions include but are not limited to, dishwashing compositions, laundry detergent compositions, fabric softening compositions, fabric enhancing compositions, fabric freshening compositions, laundry pre-wash, laundry pretreat, laundry additives, spray products, dry cleaning agent or composition, laundry rinse additive, wash additive, post-rinse fabric treatment, ironing aid, hard surface cleaning compositions, unit dose formulation, delayed delivery formulation, detergent contained on or in a porous substrate or nonwoven sheet, and other suitable forms that may be apparent to one skilled in the art in view of the teachings herein. Such compositions may be used as a pre-cleaning treatment, a post-cleaning treatment, or may be added during the rinse or wash cycle of the cleaning process. The cleaning compositions may have a form selected from liquid, powder, single-phase or multi-phase unit dose or pouch form, tablet, gel, paste, bar, or flake. Preferably the composition is for manual-washing. Preferably, the cleaning composition of the present invention is a dishwashing detergent. Preferably the composition is in the form of a liquid.

As used herein the term "fragment" means an amino acid sequence of at least 30, 60, 100, 150 contiguous amino acids of the reference sequences or any integer there between.

As used herein the term "improving suds longevity" means an increase in the duration of visible suds in a washing process cleaning soiled articles using the composition comprising one or more subclass EAS hydrophobins, compared with the suds longevity provided by the same composition and process in the absence of the subclass EAS hydrophobins.

As used herein, the term "next time cleaning benefit" means the surface to be cleaned could be treated with a composition which would assist in easier removal of soil and/or stains during subsequent cleaning.

As used herein, the term "soiled surfaces" refers non-specifically to any type of flexible material consisting of a network of natural or artificial fibers, including natural, artificial, and synthetic fibers, such as, but not limited to, cotton, linen, wool, polyester, nylon, silk, acrylic, and the like, as well as various blends and combinations. Soiled surfaces may further refer to any type of hard surface, including natural, artificial, or synthetic surfaces, such as, but not limited to, tile, granite, grout, glass, composite, vinyl, hardwood, metal, cooking surfaces, plastic, and the like, as well as blends and combinations, as well as dishware. Key targeted soiled surfaces by this application are soiled dishware.

As used herein, the term "variant" of the subclass EAS hydrophobins means an amino acid sequence when the subclass EAS hydrophobin is modified by, or at, one or more amino acids (for example 1, 2, 3, 4, 5, 6, 7, 8, 9 or 10 or more amino acid modifications) selected from substitutions, insertions, deletions and combinations thereof. The variant may have "conservative" substitutions, wherein a substituted amino acid has similar structural or chemical properties to the amino acid that replaces it, for example, replacement of leucine with isoleucine. A variant may have "non-conservative" changes, for example, replacement of a glycine with a tryptophan. Variants may also include sequences with amino acid deletions or insertions, or both. Guidance in determining which amino acid residues may be substituted, inserted, or deleted without abolishing the activity of the protein may be found using computer programs well known in the art. Variants may also include truncated forms derived from a wild-type subclass EAS hydrophobin, such as for example, a protein with a truncated N-terminus. Variants may also include forms derived by adding an extra amino acid sequence to a wild-type protein, such as for example, an N-terminal tag, a C-terminal tag or an insertion in the middle of the protein sequence.

As used herein, the term "water hardness" or "hardness" means uncomplexed cation ions (*i.e.,* $Ca^{2+}$ or $Mg^{2+}$) present in water that have the potential to precipitate with anionic surfactants or other anionic actives in the cleaning composition under alkaline conditions, and thereby diminishing the surfactancy and cleaning capacity of surfactants. Further, the terms "high water hardness" and "elevated water hardness" can be used interchangeably and are relative terms for the purposes of the present invention, and are intended to include, but not limited to, a hardness level containing at least 12 grams of calcium ion per gallon water (gpg, "American grain hardness" units).

## Cleaning Composition

The present invention envisages a cleaning composition, preferably a hand dishwashing cleaning composition, comprising a specific surfactant system and one or more subclass EAS hydrophobins. The composition of the invention provides very good suds duration especially in presence of fatty and/or oily soils. The invention also envisages a method of hand dishwashing and use of the composition for prolonging suds duration.

A preferred cleaning composition is a manual dishwashing composition, preferably in liquid form. It typically contains from 30% to 95%, preferably from 40% to 90%, more preferably from 50% to 85% by weight of the composition of a liquid carrier in which the other essential and optional components are dissolved, dispersed or suspended. One preferred component of the liquid carrier is water.

Preferably the pH of the cleaning composition of the invention, measured as a 10% product concentration in demineralized water at 20°C, is adjusted to between 3 and 14, more preferably between 4 and 13, more preferably between 6 and 12 and most preferably between 8 and 10. The pH of the cleaning composition can be adjusted using pH modifying ingredients known in the art.

## Hydrophobins and Subclass EAS Hydrophobins

The cleaning composition in accordance with the present invention comprises one or more subclass EAS hydrophobins. Hydrophobins are proteins of fungal origin that play multiple roles in the growth and development of filamentous fungi (*see* Wosten, H. A. B. (2001), Annu. Rev. Microbiol., 55: 625-646). For example, they are involved in the formation of aerial structures and in the attachment of hyphae to hydrophobic surfaces. The mechanisms by which

hydrophobins perform their function are based on their property of self-assembling at hydrophobic-hydrophilic interfaces into an amphipathic film.

In this specification, "hydrophobins" are polypeptides capable of self-assembly at a hydrophilic / hydrophobic interface, and comprise the following sequence:

$(Y_1)_n$-$B_1$-$(X_1)_a$-$B_2$-$(X_2)_b$-$B_3$-$(X_3)_c$-$B_4$-$(X_4)_d$-$B_5$-$(X_5)_e$-$B_6$-$(X_6)_f$-$B_7$-$(X_7)_g$-$B_8$-$(Y_2)_m$

wherein: m and n are independently 0 to 2000; $B_1$, $B_2$, $B_3$, $B_4$, $B_5$, $B_6$, $B_7$ and $B_8$ are each independently amino acids selected from Cys, Leu, Ala, Pro, Ser, Thr, Met or Gly, at least 6 of the residues $B_1$through $B_8$ being Cys; $X_1$, $X_2$, $X_3$, $X_4$, $X_5$, $X_6$, $X_7$,$Y_1$ and $Y_2$ independently represent any amino acid; a is 1 to 50; b is 0 to 5; c is 1 to 100; d is 1 to 100; e is 1 to 50; f is 0 to 5; g is 1 to 100; m is 0 to 100; and n is 0 to 100.

Traditionally, hydrophobins have been classified into class I or class II based on structural and physical parameters including solubility. As described herein, hydrophobins self-assemble at an interface (especially a water/air interface) into amphipathic interfacial films. The assembled amphipathic films of class I hydrophobins are generally resolubilized only in strong acids (typically those having a pKa of lower than 4, such as formic acid or trifluoroacetic acid), whereas those of class II are soluble in a wider range of solvents.

Hydrophobins of classes I and II may also be distinguished by the hydrophobicity/hydrophilicity of a number of regions of the hydrophobin proteins. The relative hydrophobicity/hydrophilicity of the various regions of the hydrophobin proteins can be established by comparing the hydropathy pattern of the hydrophobin using the method set out in Kyte and Doolittle, J. Mol. Biol., 1982, 157, 105-132. For class II hydrophobins, the region between the residues $B_3$ and $B_4$, *i.e.,* the moiety $(X_3)_c$, is predominantly hydrophobic. In contrast, for class I hydrophobins, the region between the residues $B_3$ and $B_4$, *i.e.,* the group $(X_3)_c$, is predominantly hydrophilic. Alternatively the region between the residues $B_7$ and $B_8$, *i.e.,* the moiety $(X_7)_g$, is predominantly hydrophobic for class II hydrophobins, while being predominantly hydrophilic for class I hydrophobins.

As part of the current invention, a sequence similarity network of hydrophobins was generated using EFI - Enzyme Similarity Tool Ver 2.0 (*http://efi.igb.illinois.edu/efi-est/*), allowing the identification of not only the two major traditional classes of hydrophobins (class I and class II), but also several subclasses including a group comprising EAS (SEQ ID NO: 1, obtainable from the fungus *Neurospora crassa*) and wild-type variants with high homology to EAS, referred herein as subclass EAS (see Figure 1).

The compositions of the current invention comprise one or more subclass EAS hydrophobins, whereas other hydrophobins are not part of the invention. As used herein, "subclass EAS hydrophobins" refer to any hydrophobins with at least 45% amino acid sequence identity compared to *Neurospora crassa* EAS (SEQ ID NO: 1). Besides *Neurospora crassa* EAS, subclass EAS hydrophobins comprises hydrophobins produced by *Neurospora tetrasperma* (*e.g.,* SEQ ID NO: 2, SEQ ID NO: 4), *Neurospora discreta* (*e.g.,* SEQ ID NO: 3), *Neurospora sitophila* (*e.g.,* SEQ ID NO: 5), *Neurospora terrícola* (*e.g.,* SEQ ID NO: 6), *Neurospora lineolata* (*e.g.,* SEQ ID NO: 7), *Neurospora intermedia, Neurospora africana, Neurospora dodgei,* and *Sordaria macrospora* (*e.g.,* SEQ ID NO: 8), (*see* Winefield, R. D., et al. (2007)., Fungal Genet. Biol. 44(4): 250-257).

Unexpectedly, the Applicants found that subclass EAS hydrophobins are able to increase sudsing in the presence of a specific surfactant system. Not wishing to be bound by theory, the Applicants believe that the increased sudsing benefits are due to the specific amino acid sequences and/or protein structures enhancing the adsorption at the interface between two phases (oil/water or air/water). Similar benefits are not observed when the cleaning compositions comprise class I hydrophobins different to subclass EAS hydrophobins. Furthermore, the Applicants have discovered that compositions containing subclass EAS hydrophobins have good grease cleaning and emulsification profile without negatively impacting sudsing.

Accordingly, a cleaning composition of the present invention comprises: a) from 1 wt% to 60 wt%, preferably from 5 wt% to 50 wt%, by weight of the composition of a surfactant system comprising one or more anionic surfactants and one or more co-surfactants selected from the group consisting of amphoteric surfactant, zwitterionic surfactant, and mixtures thereof; and b) from 0.001 wt% to 5 wt%, preferably from 0.1 wt% to 1 wt%, by weight of the composition, based on active protein, of one or more subclass EAS hydrophobins.

Preferably the subclass EAS hydrophobins have at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one or more reference sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 11, preferably to SEQ ID NO: 9.

The invention also includes subclass EAS hydrophobins variants. For example, subclass EAS hydrophobins variants, as used herein, include a sequence resulting when a wild-type protein is modified by, or at, one or more amino acids (for example 1, 2, 5 or 10 amino acids). The invention also includes subclass EAS hydrophobins variants in the form of truncated forms derived from a wild-type subclass EAS hydrophobin, such as a wild-type subclass EAS hydrophobin with a truncated N-terminus or a truncated C-terminus.

The majority of subclass EAS hydrophobins are predicted to include an N-terminal signal peptide that is likely removed upon secretion by the native organisms. Preferably the subclass EAS hydrophobin variants of the present invention are without the N-terminal signal peptide. For example, SEQ ID NO: 9 is a variant of the full length wild-type *Neurospora crassa* EAS (SEQ ID NO: 1) without the N-terminal signal peptide. Bioinformatic tools, such as SignalP version 4.1 (Petersen TN., Brunak S., von Heijne G. and Nielsen H. (2011), Nature Methods, 8:785-786), can be used to predict the existence and length of such signal peptides.

The invention also includes variants derived by adding an extra amino acid sequence to a wild-type protein, such as for example, an N-terminal tag, a C-terminal tag or an insertion in the middle of the protein sequence. Non-limiting examples of tags are maltose binding protein (MBP) tag, glutathione S-transferase (GST) tag, thioredoxin (Trx) tag, His-tag, ubiquitin-tag, and any other tags known by those skilled in art. Tags can be used to improve solubility and expression levels during fermentation or as a handle for enzyme purification. For example, His-Ubi-EAS (SEQ ID NO: 11) is a variant of EAS (SEQ ID NO: 9) including an N-terminal His tag, an ubiquitin tag, and a TEV protease cleavage site.

It is important that variants of subclass EAS hydrophobins retain or preferably improve the ability of the wild-type proteins to adsorb at an interface and to stabilize that interface. Some performance drop in a given property of subclass EAS hydrophobins variants may of course be tolerated, but the subclass EAS hydrophobins variants should retain or preferably improve suitable properties for the relevant application for which they are intended. For instance, screening of variants of one of the wild-types can be used to identify whether they retain or improve appropriate properties.

Suitable examples of subclass EAS hydrophobins variants include one conservative substitution in the peptide, such as a conservative substitution in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 11.

Other suitable examples of subclass EAS hydrophobins variants include 10 or fewer conservative substitutions are included in the peptide, such as five or fewer. The subclass EAS hydrophobins of the present invention may therefore include 1, 2, 3, 4, 5, 6, 7, 8, 9, 10 or more conservative substitutions. The subclass EAS hydrophobins can be produced to contain one or more conservative substitutions by manipulating the nucleotide sequence that encodes that peptide using, for example, standard procedures such as site-directed mutagenesis or PCR. Alternatively, the subclass EAS hydrophobins can be produced to contain one or more conservative substitutions by using peptide synthesis methods, for example, as known in the art.

Examples of amino acids which may be substituted for an original amino acid in a subclass EAS hydrophobin and which are regarded as conservative substitutions include: Ser for Ala; Lys for Arg; Gln or His for Asn; Glu for Asp; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

Preferably the subclass EAS hydrophobins of the invention may comprise variants of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 11, wherein one or more cysteine residues are substituted by another amino acid.

Preferably the subclass EAS hydrophobins of the present invention may comprise variants of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 11, wherein a short amino acid sequence containing two cysteine residues is added at the C-terminus or at least two residues are modified to cysteines. These cysteine residues can allow the subclass EAS hydrophobins to form multimers *(i.e.,* dimers, tetramers, hexamers and potentially higher order oligomers) in solution due to the formation of disulfide bonds between the cysteine residues of adjacent subclass EAS hydrophobins variants.

The subclass EAS hydrophobins of the present invention may also cover fragments of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 11. Preferably the subclass EAS hydrophobins fragments can adsorb to an interface and stabilize that interface.

The subclass EAS hydrophobins can be modified by a variety of chemical techniques to produce derivatives having essentially the same or even improved activity as the unmodified peptides, and optionally having other desirable properties. For example, carboxylic acid groups of the protein, whether carboxyl-terminal or side chain, may be provided in the form of a salt of a pharmaceutically-acceptable cation or esterified, for example to form a $C_1$-$C_6$ alkyl ester, or converted to an amide, for example of formula $CONR_1R_2$ wherein $R_1$ and $R_2$ are each independently H or $C_1$-$C_6$ alkyl, or combined to form a heterocyclic ring, such as a 5- or 6-membered ring. Amino groups of the peptide, whether amino-terminal or side chain, may be in the form of a pharmaceutically-acceptable acid addition salt, such as the HCl, HBr, acetic, benzoic, toluene sulfonic, maleic, tartaric and other organic salts, or may be modified to $C_1$-$C_6$ alkyl or dialkyl amino or further converted to an amide. Hydroxyl groups of the peptide side chains may be converted to alkoxy or ester groups, for example $C_1$-$C_6$ alkoxy or $C_1$-$C_6$ alkyl ester, using well-recognized techniques. Phenyl and phenolic rings of the peptide side chains may be substituted with one or more halogen atoms, such as F, Cl, Br or I, or with C1-C6 alkyl, C1-C6 alkoxy, carboxylic acids and esters thereof, or amides of such carboxylic acids. Methylene groups of the peptide side chains can be extended to homologous C2-C4 alkylenes. Thiols can be protected with any one of a number of well-recognized protecting groups, such as acetamide groups. Those skilled in the art will also recognize methods for introducing cyclic structures into the subclass EAS hydrophobins of the present invention to select and provide conformational constraints to the structure that result in enhanced stability.

Identity, or homology, percentages as mentioned herein in respect of the present invention are those that can be calculated with the GAP program, obtainable from GCG (Genetics Computer Group Inc., Madison, W1, USA). Alternatively, a manual alignment can be performed.

For polypeptide sequence comparison the following settings can be used: Alignment algorithm: Needleman and Wunsch, J. Mol. Biol. 1970, 48: 443-453. As a comparison matrix for amino acid similarity the Blosum62 matrix is used (Henikoff S. and Henikoff J.G., P.N.A.S. USA 1992, 89: 10915-10919). The following gap scoring parameters are used: Gap penalty: 12, gap length penalty: 2, no penalty for end gaps.

A given sequence is typically compared against the full-length sequence of SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9, or SEQ ID NO: 11 to obtain a score.

The cleaning composition preferably comprises from 0.001 wt% to 5 wt%, preferably from 0.1 wt% to 1 wt%, by weight of said composition, based on active protein, of one or more subclass EAS hydrophobins. Preferably the subclass EAS hydrophobin has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of at least one or more reference sequence selected from the group consisting of SEQ ID NO: 1, SEQ ID NO: 2, SEQ ID NO: 3, SEQ ID NO: 4, SEQ ID NO: 5, SEQ ID NO: 7, SEQ ID NO: 8, SEQ ID NO: 9, or SEQ ID NO: 11. More preferably the subclass EAS hydrophobin has at least 50%, preferably at least 60%, preferably at least 70%, preferably at least 80%, preferably at least 85%, preferably at least 90%, preferably at least 95%, preferably at least 98% or even 100% amino acid identity as calculated over the entire length of the sequence aligned against the entire length of *Neurospora crassa* EAS (SEQ ID NO: 9).

<u>Surfactant System</u>

The detergent composition of the invention comprises from 1% to 60%, preferably from 5% to 50%, more preferably from 8% to 40%, by weight of the total composition of a specific surfactant system.

The surfactant system of the composition of the present invention comprises an anionic surfactant. Preferably, the surfactant system for the cleaning composition of the present invention comprises from 1% to 40%, preferably 6% to 35%, more preferably 8% to 30% by weight of the total composition of an anionic surfactant. The anionic surfactant can be any anionic cleaning surfactant, preferably selected from sulfate and/or sulfonate anionic surfactants. HLAS (linear alkylbenzene sulfonate) would be the most preferred sulfonate anionic surfactant. Especially preferred anionic surfactant is selected from the group consisting of alkyl sulfate, alkyl alkoxy sufate and mixtures thereof, and preferably wherein the alkyl alkoxy sulfate is an alkyl ethoxy sulfate. Preferred anionic surfactant is a combination of alkyl sulfates and alkyl ethoxy sulfates with a combined mol average ethoxylation degree of less than 5, preferably less than 3, more preferably less than 2 and more than 0.5 and an average level of branching of from 5% to 40%, more preferably from 10% to 35%, and even more preferably from 20% to 30%.

The average alkoxylation degree is the mol average alkoxylation degree of all the components of the mixture (*i.e.,* mol average alkoxylation degree) of the anionic surfactant. In the mol average alkoxylation degree calculation the weight of sulfate anionic surfactant components not having alkoxylate groups should also be included.

$$\text{Mol average alkoxylation degree} = (x1 * \text{alkoxylation degree of surfactant } 1 + x2 * \text{alkoxylation degree of surfactant } 2 + \ldots) / (x1 + x2 + \ldots)$$

wherein x1, x2, ... are the number of moles of each sulfate anionic surfactant of the mixture and alkoxylation degree is the number of alkoxy groups in each sulfate anionic surfactant.

The average level of branching is the weight average % of branching and it is defined according to the following formula:

$$\text{Weight average of branching } (\%) = [(x1 * \text{wt\% branched alcohol 1 in alcohol 1} + x2 * \text{wt\% branched alcohol 2 in alcohol 2} + \ldots) / (x1 + x2 + \ldots)] * 100$$

wherein x1, x2, ... are the weight in grams of each alcohol in the total alcohol mixture of the alcohols which were used as starting material for the anionic surfactant for the composition of the invention. In the weight average branching degree

calculation the weight of anionic surfactant components not having branched groups should also be included.

Suitable examples of commercially available sulfates include, those based on Neodol alcohols ex the Shell company, Lial - Isalchem and Safol ex the Sasol company, natural alcohols ex The Procter & Gamble Chemicals company. Suitable sulfonate surfactants for use herein include water-soluble salts of C8-C18 alkyl or hydroxyalkyl sulfonates; C11-C18 alkyl benzene sulfonates (LAS), modified alkylbenzene sulfonate (MLAS); methyl ester sulfonate (MES); and alpha-olefin sulfonate (AOS). Those also include the paraffin sulfonates may be monosulfonates and/or disulfonates, obtained by sulfonating paraffins of 10 to 20 carbon atoms. The sulfonate surfactant also include the alkyl glyceryl sulfonate surfactants.

The surfactant system of the composition of the present invention further comprises a primary co-surfactant system, wherein the primary co-surfactant system is selected from the group consisting of amphoteric surfactant, zwitterionic surfactant and mixtures thereof. Preferably, the surfactant system for the cleaning composition of the present invention comprises from 0.5% to 15%, preferably from 1% to 12%, more preferably from 2% to 10%, by weight of the total composition of a primary co-surfactant system.

Preferably the primary co-surfactant system is an amphoteric surfactant. Preferably, the primary co-surfactant system is an amine oxide surfactant, and wherein the composition comprises anionic surfactant and amine oxide surfactant in a ratio of less than 9:1, more preferably from 5:1 to 1:1, more preferably from 4:1 to 2:1, preferably from 3:1 to 2.5:1. Preferred amine oxides are alkyl dimethyl amine oxide or alkyl amido propyl dimethyl amine oxide, more preferably alkyl dimethyl amine oxide and especially coco dimethyl amino oxide. Amine oxide may have a linear or branched alkyl moiety.

Preferably the amine oxide surfactant is a mixture of amine oxides comprising a low-cut amine oxide and a mid-cut amine oxide. The amine oxide of the composition of the invention then comprises:

a) from 10% to 45% by weight of the amine oxide of low-cut amine oxide of formula R1R2R3AO wherein R1 and R2 are independently selected from hydrogen, C1-C4 alkyls or mixtures thereof, and R3 is selected from C10 alkyls or mixtures thereof; and

b) from 55% to 90% by weight of the amine oxide of mid-cut amine oxide of formula R4R5R6AO wherein R4 and R5 are independently selected from hydrogen, C1-C4 alkyls or mixtures thereof, and R6 is selected from C12-C16 alkyls or mixtures thereof

In a preferred low-cut amine oxide for use herein R3 is n-decyl. In another preferred low-cut amine oxide for use herein R1 and R2 are both methyl. In an especially preferred low-cut amine oxide for use herein R1 and R2 are both methyl and R3 is n-decyl.

Preferably, the amine oxide comprises less than 5%, more preferably less than 3%, by weight of the amine oxide of an amine oxide of formula R7R8R9AO wherein R7 and R8 are selected from hydrogen, C1-C4 alkyls and mixtures thereof and wherein R9 is selected from C8 alkyls and mixtures thereof. Compositions comprising R7R8R9AO tend to be unstable and do not provide very suds mileage.

Preferably the primary co-surfactant system is a zwitterionic surfactant. Suitable examples of zwitterionic surfactants include betaines, such as alkyl betaines, alkylamidobetaine, amidazoliniumbetaine, sulfobetaine (INCI Sultaines) as well as the Phosphobetaine and preferably meets formula (I):

RI-[CO-X (CH2)n]x-N+(R2)(R3)-(CH2)m-[CH(OH)-CH2]y-Y- (I)

wherein

R1 is a saturated or unsaturated C6-22 alkyl residue, preferably C8-18 alkyl residue, in particular a saturated C10-16 alkyl residue, for example a saturated C12-14 alkyl residue;

X is NH, NR4 with C1-4 Alkyl residue R4, O or S;

n is a number from 1 to 10, preferably 2 to 5, in particular 3;

x is 0 or 1, preferably 1;

R2 and R3 are independently a C1-4 alkyl residue, potentially hydroxy substituted such as a hydroxyethyl, preferably a methyl;

m is a number from 1 to 4, in particular 1, 2 or 3;

y is 0 or 1; and

Y is COO, SO3, OPO(OR5)O or P(O)(OR5)O, whereby R5 is a hydrogen atom H or a C1-4 alkyl residue.

Preferred betaines are the alkyl betaines of the formula (Ia), the alkyl amido propyl betaine of the formula (Ib), the Sulfo betaines of the formula (Ic), and the Amido sulfobetaine of the formula (Id);

R1-N+(CH3)2-CH2COO- (Ia)

R1-CO-NH(CH2)3-N+(CH3)2-CH2COO- (Ib)

R1-N+(CH3)2-CH2CH(OH)CH2SO3- (Ic)

R1-CO-NH-(CH2)3-N+(CH3)2-CH2CH(OH)CH2SO3- (Id)

in which R1 has the same meaning as in formula (I). Particularly preferred betaines are the Carbobetaine [wherein Y-=COO-], in particular the Carbobetaine of the formula (Ia) and (Ib), more preferred are the Alkylamidobetaine of the formula (Ib). A preferred betaine is, for example, Cocoamidopropylbetaine.

Preferably the surfactant system of the composition of the present invention further comprises from 0.1% to 10% by weight of the total composition of a secondary co-surfactant system preferably comprising a non-ionic surfactant. Suitable non-ionic surfactants include the condensation products of aliphatic alcohols with from 1 to 25 moles of ethylene oxide. The alkyl chain of the aliphatic alcohol can either be straight or branched, primary or secondary, and generally contains from 8 to 22 carbon atoms. Particularly preferred are the condensation products of alcohols having an alkyl group containing from 10 to 18 carbon atoms, preferably from 10 to 15 carbon atoms with from 2 to 18 moles, preferably 2 to 15, more preferably 5-12 of ethylene oxide per mole of alcohol. Highly preferred non-ionic surfactants are the condensation products of guerbet alcohols with from 2 to 18 moles, preferably 2 to 15, more preferably 5-12 of ethylene oxide per mole of alcohol. Preferably, the non-ionic surfactants are an alkyl ethoxylated surfactants, preferably comprising from 9 to 15 carbon atoms in its alkyl chain and from 5 to 12 units of ethylene oxide per mole of alcohol. Other suitable non-ionic surfactants for use herein include fatty alcohol polyglycol ethers, alkylpolyglucosides and fatty acid glucamides, preferably alkylpolyglucosides. Preferably the alkyl polyglucoside surfactant is a C8-C16 alkyl polyglucoside surfactant, preferably a C8-C14 alkyl polyglucoside surfactant, preferably with an average degree of polymerization of between 0.1 and 3, more preferably between 0.5 and 2.5, even more preferably between 1 and 2. Most preferably the alkyl polyglucoside surfactant has an average alkyl carbon chain length between 10 and 16, preferably between 10 and 14, most preferably between 12 and 14, with an average degree of polymerization of between 0.5 and 2.5 preferably between 1 and 2, most preferably between 1.2 and 1.6. C8-C16 alkyl polyglucosides are commercially available from several suppliers (*e.g.*, Simusol® surfactants from Seppic Corporation; and Glucopon® 600 CSUP, Glucopon® 650 EC, Glucopon® 600 CSUP/MB, and Glucopon® 650 EC/MB, from BASF Corporation). Preferably, the composition comprises the anionic surfactant and the non-ionic surfactant in a ratio of from 2:1 to 50:1, preferably 2:1 to 10:1.

Enzymes

Preferred compositions of the invention may comprise one or more enzymes selected from the group consisting of amylases, lipases, proteases, cellulases, lipoxygenases, diol synthases, and mixtures thereof. When present in a composition, the aforementioned enzymes may be present at levels from 0.00001 wt% to 2 wt%, from 0.0001 wt% to 1 wt% or from 0.001 wt% to 0.5 wt% by weight of the composition, based on active protein.

Enzyme Stabilizer

Preferably the composition of the invention comprises an enzyme stabilizer. Suitable enzyme stabilizers may be selected from the group consisting of (a) univalent, bivalent and/or trivalent cations preferably selected from the group of inorganic or organic salts of alkaline earth metals, alkali metals, aluminum, iron, copper and zinc, preferably alkali metals and alkaline earth metals, preferably alkali metal and alkaline earth metal salts with halides, sulfates, sulfites, carbonates, hydrogencarbonates, nitrates, nitrites, phosphates, formates, acetates, propionates, citrates, maleates, tartrates, succinates, oxalates, lactates, and mixtures thereof. Preferably the salt is selected from the group consisting of sodium chloride, calcium chloride, potassium chloride, sodium sulfate, potassium sulfate, sodium acetate, potassium acetate, sodium formate, potassium formate, calcium lactate, calcium nitrate and mixtures thereof. Most preferred are salts selected from the group consisting of calcium chloride, potassium chloride, potassium sulfate, sodium acetate, potassium acetate, sodium formate, potassium formate, calcium lactate, calcium nitrate, and mixtures thereof, and in particular potassium salts selected from the group of potassium chloride, potassium sulfate, potassium acetate, potassium formate, potassium propionate, potassium lactate and mixtures thereof. Most preferred are potassium acetate and potassium chloride. Preferred calcium salts are calcium formate, calcium lactate and calcium nitrate including calcium nitrate tetrahydrate. Calcium and sodium formate salts may be preferred. These cations are present at at least 0.01 wt%, preferably at least 0.03 wt%, more preferably at least 0.05 wt%, most preferably at least 0.25 wt% up to 2 wt% or even up to 1 wt% by weight of the total composition. These salts are formulated from 0.1 to 5 wt%, preferably from 0.2 to 4 wt%, more preferably from 0.3 to 3 wt%, most preferably from 0.5 to 2 wt% relative to the total weight of the

composition. Further enzyme stabilizers can be selected from the group (b) carbohydrates selected from the group consisting of oligosaccharides, polysaccharides and mixtures thereof, such as a monosaccharide glycerate as described in WO201219844; (c) mass efficient reversible protease inhibitors selected from the group consisting of phenyl boronic acid and derivatives thereof, preferably 4-formyl phenylboronic acid; (d) alcohols such as 1,2-propane diol, propylene glycol; (e) peptide aldehyde stabilizers such as tripeptide aldehydes such as Cbz-Gly-Ala-Tyr-H, or disubstituted alaninamide; (f) carboxylic acids such as phenyl alkyl dicarboxylic acid as described in WO2012/19849 or multiply substituted benzyl carboxylic acid comprising a carboxyl group on at least two carbon atoms of the benzyl radical such as described in WO2012/19848, phthaloyl glutamine acid, phthaloyl asparagine acid, aminophthalic acid and/or an oligoamino-biphenyl-oligocarboxylic acid; and (g) mixtures thereof.

Salt

The composition of the present invention may optionally comprise from 0.01% to 3%, preferably from 0.05% to 2%, more preferably from 0.2% to 1.5%, or most preferably 0.5% to 1%, by weight of the total composition of a salt, preferably a monovalent, divalent inorganic salt or a mixture thereof, preferably sodium chloride. Most preferably the composition alternatively or further comprises a multivalent metal cation in the amount of from 0.01 wt% to 3 wt%, preferably from 0.05% to 2%, more preferably from 0.2% to 1.5%, or most preferably 0.5% to 1% by weight of said composition, preferably said multivalent metal cation is magnesium, aluminium, copper, calcium or iron, more preferably magnesium, most preferably said multivalent salt is magnesium chloride. Without wishing to be bound by theory, it is believed that use of a multivalent cation helps with the formation of protein/ protein, surfactant/ surfactant or hybrid protein/ surfactant network at the oil water and air water interface that is strengthening the suds.

Carbohydrates

Preferably the composition of the present invention comprises one or more carbohydrates selected from the group comprising O-glycan, N-glycan, and mixtures thereof. Preferably the cleaning composition further comprises one or more carbohydrates selected from the group comprising derivatives of glucose, mannose, lactose, galactose, allose, altrose, gulose, idose, talose, fucose, fructose, sorbose, tagatose, psicose, arabinose, ribose, xylose, lyxose, ribulose, and xylulose. More preferably the cleaning composition comprises one or more carbohydrates selected from the group of $\alpha$-glucans and $\beta$-glucans. Glucans are polysaccharides of D-glucose monomers, linked by glycosidic bonds. Non-limiting examples of $\alpha$-glucans are dextran, starch, floridean starch, glycogen, pullulan, and their derivatives. Non-limiting examples of $\beta$-glucans are cellulose, chrysolaminarin, curdlan, laminarin, lentinan, lichenin, oat beta-glucan, pleuran, zymosan, and their derivatives.

Hydrotrope

The composition of the present invention may optionally comprise from 1% to 10%, or preferably from 0.5% to 10%, more preferably from 1% to 6%, or most preferably from 0.1% to 3%, or combinations thereof, by weight of the total composition of a hydrotrope, preferably sodium cumene sulfonate. Other suitable hydrotropes for use herein include anionic-type hydrotropes, particularly sodium, potassium, and ammonium xylene sulfonate, sodium, potassium and ammonium toluene sulfonate, sodium potassium and ammonium cumene sulfonate, and mixtures thereof, as disclosed in U.S. Patent 3,915,903. Preferably the composition of the present invention is isotropic. An isotropic composition is distinguished from oil-in-water emulsions and lamellar phase compositions. Polarized light microscopy can assess whether the composition is isotropic. See e.g., The Aqueous Phase Behaviour of Surfactants, Robert Laughlin, Academic Press, 1994, pp. 538-542. Preferably an isotropic composition is provided. Preferably the composition comprises 0.1% to 3% by weight of the total composition of a hydrotrope, preferably wherein the hydrotrope is selected from sodium, potassium, and ammonium xylene sulfonate, sodium, potassium and ammonium toluene sulfonate, sodium potassium and ammonium cumene sulfonate, and mixtures thereof.

Organic Solvent

The composition of the present invention may optionally comprise an organic solvent. Suitable organic solvents include C4-14 ethers and diethers, polyols, glycols, alkoxylated glycols, C6-C16 glycol ethers, alkoxylated aromatic alcohols, aromatic alcohols, aliphatic linear or branched alcohols, alkoxylated aliphatic linear or branched alcohols, alkoxylated C1-C5 alcohols, C8-C14 alkyl and cycloalkyl hydrocarbons and halohydrocarbons, and mixtures thereof. Preferably the organic solvents include alcohols, glycols, and glycol ethers, alternatively alcohols and glycols. The composition comprises from 0% to less than 50%, preferably from 0.01% to 25%, more preferably from 0.1% to 10%, or most preferably from 0.5% to 5%, by weight of the total composition of an organic solvent, preferably an alcohol, more

preferably an ethanol, a polyalkyleneglycol, more preferably polypropyleneglycol, and mixtures thereof.

Amphiphilic Polymer

The composition of the present invention may further comprise from 0.01% to 5%, preferably from 0.05% to 2%, more preferably from 0.07% to 1% by weight of the total composition of an amphiphilic polymer selected from the groups consisting of amphiphilic alkoxylated polyalkyleneimine and mixtures thereof, preferably an amphiphilic alkoxylated polyalkyleneimine.

Preferably, the amphiphilic alkoxylated polyalkyleneimine is an alkoxylated polyethyleneimine polymer comprising a polyethyleneimine backbone having average molecular weight range from 100 to 5,000, preferably from 400 to 2,000, more preferably from 400 to 1,000 Daltons and the alkoxylated polyethyleneimine polymer further comprising:

(i) one or two alkoxylation modifications per nitrogen atom by a polyalkoxylene chain having an average of 1 to 50 alkoxy moieties per modification, wherein the terminal alkoxy moiety of the alkoxylation modification is capped with hydrogen, a C1-C4 alkyl or mixtures thereof;
(ii) an addition of one C1-C4 alkyl moiety and one or two alkoxylation modifications per nitrogen atom by a polyalkoxylene chain having an average of 1 to 50 alkoxy moieties per modification wherein the terminal alkoxy moiety is capped with hydrogen, a C1-C4 alkyl or mixtures thereof; or
(iii) a combination thereof; and

wherein the alkoxy moieties comprises ethoxy (EO) and/or propxy (PO) and/or butoxy (BO) and wherein when the alkoxylation modification comprises EO it also comprises PO or BO.

Preferred amphiphilic alkoxylated polyethyleneimine polymers comprise EO and PO groups within their alkoxylation chains, the PO groups preferably being in terminal position of the alkoxy chains, and the alkoxylation chains preferably being hydrogen capped. Hydrophilic alkoxylated polyethyleneimine polymers solely comprising ethoxy (EO) units within the alkoxylation chain could also optionally be formulated within the scope of this invention.

For example, but not limited to, below is shown possible modifications to terminal nitrogen atoms in the polyethyleneimine backbone where R represents an ethylene spacer and E represents a C1-C4 alkyl moiety and X- represents a suitable water soluble counterion.

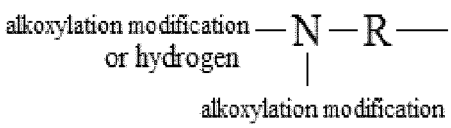

or

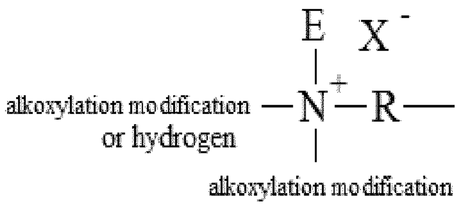

Also, for example, but not limited to, below is shown possible modifications to internal nitrogenatoms in the polyethyleneimine backbone where R represents an ethylene spacer and E represents a $C_1$-$C_4$ alkyl moiety and X- represents a suitable water soluble counterion.

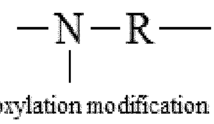

or

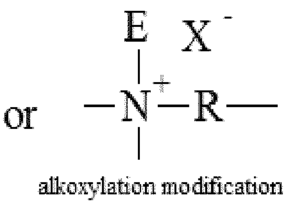

The alkoxylation modification of the polyethyleneimine backbone consists of the replacement of a hydrogen atom by a polyalkoxylene chain having an average of 1 to 50 alkoxy moieties, preferably from 20 to 45 alkoxy moieties, most preferably from 30 to 45 alkoxy moieties. The alkoxy moieties are selected from ethoxy (EO), propoxy (PO),butoxy (BO), and mixtures thereof. Alkoxy moieties solely comprising ethoxy units are outside the scope of the invention though. Preferably, the polyalkoxylene chain is selected from ethoxy/propoxy block moieties. More preferably, the polyalkoxylene chain is ethoxy/propoxy block moieties having an average degree of ethoxylation from 3 to 30 and an average degree of propoxylation from 1 to 20, more preferably ethoxy/propoxy block moieties having an average degree of ethoxylation from 20 to 30 and an average degree of propoxylation from 10 to 20.

More preferably the ethoxy/propoxy block moieties have a relative ethoxy to propoxy unit ratio between 3 to 1 and 1 to 1, preferably between 2 to 1 and 1 to 1. Most preferably the polyalkoxylene chain is the ethoxy/propoxy block moieties wherein the propoxy moiety block is the terminal alkoxy moiety block.

The modification may result in permanent quaternization of the polyethyleneimine backbone nitrogen atoms. The degree of permanent quaternization may be from 0% to 30% of the polyethyleneimine backbone nitrogen atoms. It is preferred to have less than 30% of the polyethyleneimine backbone nitrogen atoms permanently quaternized. Most preferably the degree of quaternization is 0%.

A preferred polyethyleneimine has the general structure of Formula (II):

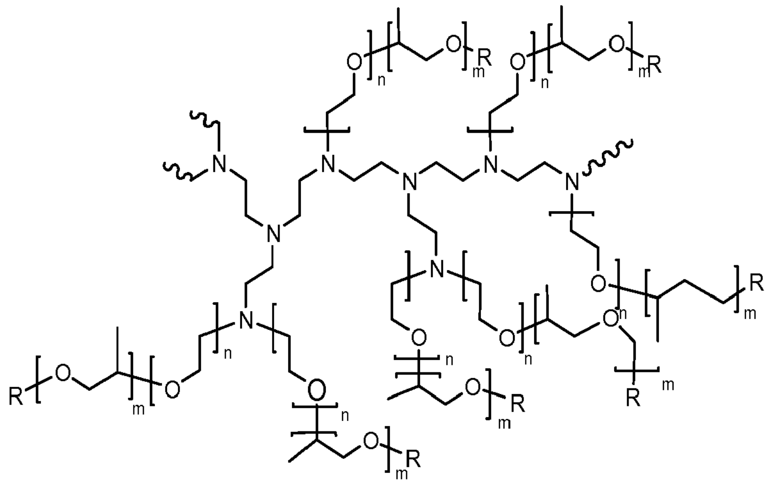

(II)

wherein the polyethyleneimine backbone has a weight average molecular weight of 600, n of formula (II) has an average of 10, m of formula (II) has an average of 7 and R of formula (II) is selected from hydrogen, a $C_1$-$C_4$ alkyl and mixtures thereof, preferably hydrogen. The degree of permanent quaternization of formula (II) may be from 0% to 22% of the polyethyleneimine backbone nitrogen atoms. The molecular weight of this polyethyleneimine preferably is between 10,000 and 15,000.

An alternative polyethyleneimine has the general structure of Formula (II) but wherein the polyethyleneimine backbone has a weight average molecular weight of 600, n of Formula (II) has an average of 24, m of Formula (II) has an average of 16 and R of Formula (II) is selected from hydrogen, a $C_1$-$C_4$ alkyl and mixtures thereof, preferably hydrogen. The degree of permanent quaternization of Formula (II) may be from 0% to 22% of the polyethyleneimine backbone nitrogen atoms. The molecular weight of this polyethyleneimine preferably is between 25,000 and 30,000.

Most preferred polyethyleneimine has the general structure of Formula (II) wherein the polyethyleneimine backbone has a weight average molecular weight of 600, n of Formula (II) has an average of 24, m of Formula (II) has an average of 16 and R of Formula (II) is hydrogen. The degree of permanent quaternization of Formula (II) is 0% of the polyethyleneimine backbone nitrogen atoms. The molecular weight of this polyethyleneimine preferably is from 25,000 to 30,000, most preferably 28,000.

These polyethyleneimines can be prepared, for example, by polymerizing ethyleneimine in the presence of a catalyst such as carbon dioxide, sodium bisulfite, sulfuric acid, hydrogen peroxide, hydrochloric acid, acetic acid, and the like, as described in more detail in PCT Publication No. WO 2007/135645.

Chelant

The detergent composition herein can comprise a chelant at a level of from 0.1% to 20%, preferably from 0.2% to 5%, more preferably from 0.2% to 3% by weight of total composition.

As commonly understood in the detergent field, chelation herein means the binding or complexation of a bi- or multidentate ligand. These ligands, which are often organic compounds, are called chelants, chelators, chelating agents, and/or sequestering agent. Chelating agents form multiple bonds with a single metal ion. Chelants, are chemicals that form soluble, complex molecules with certain metal ions, inactivating the ions so that they cannot normally react with other elements or ions to produce precipitates or scale, or forming encrustations on soils turning them harder to be removed. The ligand forms a chelate complex with the substrate. The term is reserved for complexes in which the metal ion is bound to two or more atoms of the chelant.

Preferably, the composition of the present invention comprises one or more chelant, preferably selected from the group comprising carboxylate chelants, amino carboxylate chelants, amino phosphonate chelants such as MGDA

(methylglycine-N,N-diacetic acid), GLDA (glutamic-N,N- diacetic acid), and mixtures thereof.

Suitable chelating agents can be selected from the group consisting of amino carboxylates, amino phosphonates, polycarboxylate chelating agents and mixtures thereof.

Other chelants include homopolymers and copolymers of polycarboxylic acids and their partially or completely neutralized salts, monomeric polycarboxylic acids and hydroxycarboxylic acids and their salts. Suitable polycarboxylic acids are acyclic, alicyclic, heterocyclic and aromatic carboxylic acids, in which case they contain at least two carboxyl groups which are in each case separated from one another by, preferably, no more than two carbon atoms. A suitable hydroxycarboxylic acid is, for example, citric acid. Another suitable polycarboxylic acid is the homopolymer of acrylic acid. Preferred are the polycarboxylates end capped with sulfonates.

Adjunct Ingredients

The cleaning composition herein may optionally comprise a number of other adjunct ingredients such as builders (*e.g.*, preferably citrate), cleaning solvents, cleaning amines, conditioning polymers, cleaning polymers, surface modifying polymers, soil flocculating polymers, structurants, emollients, humectants, skin rejuvenating actives, enzymes, carboxylic acids, scrubbing particles, bleach and bleach activators, perfumes, malodor control agents, pigments, dyes, opacifiers, beads, pearlescent particles, microcapsules, inorganic cations such as alkaline earth metals such as Ca/Mg-ions, antibacterial agents, preservatives, viscosity adjusters (*e.g.,* salt such as NaCl, and other mono-, di- and trivalent salts) and pH adjusters and buffering means (*e.g*., carboxylic acids such as citric acid, HCl, NaOH, KOH, alkanolamines, phosphoric and sulfonic acids, carbonates such as sodium carbonates, bicarbonates, sesquicarbonates, borates, silicates, phosphates, imidazole and alike).

Method of Washing

In another aspect of the invention is directed to a method of washing dishware with the composition of the present invention. The method comprises contacting a cleaning composition with a surface; wherein said cleaning composition comprises a surfactant system and one or more subclass EAS hydrophobins according to the present invention. As such, the composition herein will be applied in its diluted form to the dishware. Soiled surfaces *e.g.* dishes are contacted with an effective amount, typically from 0.5 mL to 20 mL (per 25 dishes being treated), preferably from 3mL to 10 mL, of the detergent composition of the present invention, preferably in liquid form, diluted in water. The actual amount of detergent composition used will be based on the judgment of the user, and will typically depend upon factors such as the particular product formulation of the composition, including the concentration of active ingredients in the composition, the number of soiled dishes to be cleaned, the degree of soiling on the dishes, and the like. Generally, from 0.01 mL to 150 mL, preferably from 3 mL to 40 mL of a liquid detergent composition of the invention is combined with from 2,000 mL to 20,000 mL, more typically from 5,000 mL to 15,000 mL of water in a sink having a volumetric capacity in the range of from 1,000 mL to 20,000 mL, more typically from 5,000 mL to 15,000 mL. The soiled dishes are immersed in the sink containing the diluted compositions then obtained, where contacting the soiled surface of the dish with a cloth, sponge, or similar article cleans them. The cloth, sponge, or similar article may be immersed in the detergent composition and water mixture prior to being contacted with the dish surface, and is typically contacted with the dish surface for a period of time ranged from 1 to 10 seconds, although the actual time will vary with each application and user. The contacting of cloth, sponge, or similar article to the surface is preferably accompanied by a concurrent scrubbing of the surface.

In another aspect, the invention is directed to a method of manually washing soiled articles comprising contacting a cleaning composition with a surface, wherein the composition comprises a surfactant system and one or more subclass EAS hydrophobin according to the present invention, and wherein the composition modifies the hydrophobicity of the surface as a result of the contacting step.

Another aspect of the present invention is directed to a method of improving suds longevity in a washing process for washing soiled articles, preferably dishware. The method comprises the steps of: a) delivering a cleaning composition comprising a surfactant system and one or more subclass EAS hydrophobins according to the present invention and a surfactant system to a volume of water to form a wash liquor; and b) immersing the soiled articles into said wash liquor. Preferably the subclass EAS hydrophobins are present at a concentration of 0.005 ppm to 60 ppm, preferably at a concentration of 0.02 ppm to 12 ppm, in an aqueous wash liquor during the washing process

In another aspect, the invention is directed use of one or more subclass EAS hydrophobins to provide increased suds longevity in an aqueous wash liquor during a washing process.

TEST METHODS

The following assays set forth must be used in order that the invention described and claimed herein may be

more fully understood.

Test Method 1 - Glass Vial Suds Mileage Method

The objective of the glass vial suds mileage test method is to measure the evolution of suds volume over time generated by a certain solution of detergent composition in the presence of a greasy soil, *e.g.,* olive oil. The steps of the method are as follows:

1. Test solutions are prepared by subsequently adding aliquots at room temperature of: a) 10 g of an aqueous detergent solution at specified detergent concentration and water hardness, b) 1.0 g of an aqueous protein solution at specified concentration and water hardness, and c) 0.11 g of olive oil (Bertolli®, Extra Virgin Olive Oil), into a 40 mL glass vial (dimensions: 95 mm H x 27.5 mm D). For the reference samples, the protein solutions are substituted with 1.0 mL of demineralized water. For the nil detergent samples, the 10g of aqueous detergent solution is replaced by 10 g of water at specified water hardness.
2. The test solutions are mixed in the closed test vials by stirring at room temperature for 2 minutes on a magnetic stirring plate (IKA, model # RTC B S001; VWR magnetic stirrer, catalog # 58949-012; 500 RPM), followed by manually shaking for 20 seconds with an upwards downwards movement (about 2 up and down cycles per second, +/- 30 cm up and 30 cm down).
3. Following the shaking, the test solutions in the closed vials are further stirred on a magnetic stirring plate (IKA, model # RTC B S001; VWR magnetic stirrer, catalog # 58949-012; 500 RPM) for 60 minutes inside a water bath at 46 °C to maintain a constant temperature. The samples are then shaken manually for another 20 seconds as described above and the initial suds heights (H1) are recorded with a ruler.
4. The samples are incubated for an additional 30 minutes inside the water bath at 46 °C while stirring (IKA, model # RTC B S001; VWR magnetic stirrer, catalog # 58949-012; 500 RPM), followed by manual shaking for another 20 seconds as described above. The final suds heights (H2) are recorded.
5. Protein solutions that produce larger suds heights (HI and H2), preferably combined with lower drops in suds height between H1 and H2, are more desirable.

Test Method 2 - Sink Suds Mileage Method

The evolution of the suds volume generated by a solution of a detergent composition can be determined while adding soil loads periodically as follows. A stream of hard water (15 dH) fills a sink (cylinder dimensions: 300 mm D x 288 mm H) to 4 L with a constant pressure of 4 bar. Simultaneously, an aliquot of the detergent composition (final concentration 0.12 w%) is dispensed through a pipette with a flow rate of 0.67 mL/sec at a height of 37 cm above the bottom of the sink surface. An initial suds volume is generated in the sink due to the pressure of the water. The temperature of the solution is maintained at 46 °C during the test.

After recording the initial suds volume (average suds height x sink surface area), a fixed amount of greasy soil (Composition : see Table 1,6 mL) is injected in the middle of the sink, while a paddle (dimensions: 10 cm x 5 cm, positioned in the middle of the sink at the air liquid interface at an angle of 45 degrees) rotates 20 times into the solution at 85 RPM. This step is followed immediately by another measurement of the total suds volume. The soil injecting, paddling, and measuring steps are repeated until the measured suds volume reaches a minimum level, which is set at 400 $cm^3$. The amount of soil additions needed to get to that level is recorded. The complete process is repeated a number of times and the average of the number of additions for all the replicates is calculated for each detergent composition

Finally, the suds mileage index is then calculated as: (average number of soil additions for test detergent composition) / (average number of soil additions for reference detergent composition) x 100.

Pending on the test purpose the skilled person could choose to select an alternative water hardness, solution temperature, product concentration or soil type.

Table 1 - Greasy Soil Composition

| **Ingredient** | **Weight %** |
|---|---|
| Crisco oil | 12.730 |
| Crisco shortening | 27.752 |
| Lard | 7.638 |
| Refined Rendered Edible Beef Tallow | 51.684 |

(continued)

| Ingredient | Weight % |
|---|---|
| Oleic Acid, 90% (Techn) | 0.139 |
| Palmitic Acid, 99+% | 0.036 |
| Stearic Acid, 99+% | 0.021 |

EXAMPLES

The following examples are provided to further illustrate the present invention and are not to be construed as limitations of the present invention, as many variations of the present invention are possible without departing from its spirit or scope.

Example 1a - Production of *Neurospora crassa* EAS Hydrophobin

A codon optimized gene (SEQ ID NO: 10) encoding for a variant of *Neurospora crassa* EAS, including an N-terminal His tag, ubiquitin tag, and TEV protease cleavage site (SEQ ID NO: 11), is designed and synthesized. After synthesis, the gene is subcloned into a pET30a vector for heterologous expression. The protein is expressed and purified by Genscript (Piscataway, NJ). In brief, *Escherichia coli* BL21 (DE3) cells are transformed with the recombinant plasmid and a single colony is inoculated into TB medium containing kanamycin. Cultures are incubated at 37 °C until $OD_{600}$ reaches 1.2, followed by addition of isopropyl β-D-1-thiogalactopyranoside (IPTG) (final concentration 1 mM) to induce protein expression. The culture is then incubated at 15 °C for 16 h at 200 rpm. Cells are harvested by centrifugation and the pellets are lysed by sonication. After centrifugation, the supernatant is collected and the protein is purified by one-step purification using a nickel affinity column and standard protocols known in the art. The protein is stored in a buffer containing 50 mM Tris-HCl, 150 mM NaCl, and 10% Glycerol at pH 8.0. The final protein concentration is 0.50 mg/ mL as determined by Bradford protein assay with BSA as a standard (ThermoFisher, catalog # 23236).

Example 1b - Detergent Compositions

The evolution of suds volume generated by a certain solution of detergent composition in presence of a soil, *i.e.,* olive oil or greasy soil, is followed over time under specific conditions (*e.g.,* water hardness, solution temperature, detergent concentrations, etc.). The following solutions are prepared:

A. Hard water (15 dH): 0.75 g $MgCl_2.6H_2O$(Sigma-Aldrich, catalog # M9272), 2.10 g $CaCl_2.6H_2O$ (Sigma-Aldrich, catalog # 21108), and 0.689 g $NaHCO_3$ (Sigma-Aldrich, catalog # 31437) are dissolved in 5 L of demineralized water.
B. Detergent solution of a high surfactant content detergent composition ("solution DG-HS") is prepared using Fairy Dark Green, as commercially available in the UK in Feb 2017, diluted in hard water (15 dH) prepared as above, at targeted detergent concentration of 0.12%.
C. Detergent solution of a low surfactant content detergent composition ("solution DG-LS") is prepared using Fairy Dark Green, as commercially available in the UK in Feb 2017, diluted in hard water (15 dH) prepared as above, at targeted detergent concentration of 0.06%.
D. Protein solutions: Proteins are diluted in demineralized water to the required concentration before proceeding with the suds mileage method.
E. Greasy soil: A grease soil is prepared according to the composition described in Table 1.

Example 2 - Glass Vial Suds Mileage of *Neurospora crassa* EAS Hydrophobin with Olive Oil

Inventive Composition A is an example of a cleaning composition according to the present invention, made with: a) detergent solution DG-LS (prepared as described in Example 1b), and b) diluted samples of a purified variant of *Neurospora crassa* EAS Hydrophobin (SEQ ID NO: 11) (prepared as described in Example 1a). Comparative Composition B contains the same detergent solution DG-LS in the absence of the enzyme. Comparative Composition C contains diluted samples of a purified variant of *Neurospora crassa* EAS Hydrophobin in the absence of the detergent solution DG-LS (replaced with hard water - 15 dH). The glass vial suds mileage test is performed on the compositions using olive oil as described in the test methods section (Test Method 1).

The initial (H1) and final (H2) measurements are recorded in Table 2. The % suds height drop represents the drop in suds height as measured between the initial and final time point and is calculated by the following equation:

$$\% \text{ suds height drop} = \{(H1 - H2)/H1\} * 100\%.$$

The % sud height drops are calculated for the compositions and shown in Table 2.

Table 2: Suds Mileage

| Compositions | EAS Hydrophobin Concentration in Composition [ppm] | H1 [mm] | H2 [mm] | % suds height drop H2 vs H1 |
|---|---|---|---|---|
| Inventive Composition A | 12 | 14 | 14 | 0% |
| Comparative Composition B | 0 | 13 | 9 | 31% |
| Comparative Composition C | 12 | 0 | 0 | not applicable (no suds) |

The results confirm that Inventive Composition A detergent solution comprising a variant of *Neurospora crassa* EAS Hydrophobin according to the invention (SEQ ID NO: 11) has a superior suds profile compared to Comparative Composition B solution without the *Neurospora crassa* EAS Hydrophobin protein, both in view of absolute suds height build-up as in view of sustaining the suds height in presence of greasy soil. Comparative Composition C comprising a variant of *Neurospora crassa* EAS Hydrophobin according to the invention (SEQ ID NO: 11) without the specific surfactant system produced no suds. As such a synergistic suds boost in the presence of an oily soil (*e.g.,* olive oil) between the protein and the specific surfactant system according to the invention is illustrated.

Comparative Example 3 - Glass Vial Suds Mileage of *Schizophyllum commune* SC3 with Olive Oil

Comparative Composition D is an example of a cleaning composition outside of the scope of the present invention, made with: a) detergent solution DG-LS according to the invention (prepared as described in Example 1b), and b) diluted samples of class I hydrophobin SC3 (Sigma Aldrich, catalog # 68795) from *Schizophyllum commune* (SEQ ID NO: 13) outside the scope of the invention. Comparative Composition E contains the same detergent solution DG-LS in the absence of the protein. The glass vial suds mileage test is performed using olive oil as described in the test methods section (Test Method 1). The initial (H1) and final (H2) measurements are recorded in Table 2. The % suds height drops are calculated for the compositions and are shown in Table 3.

Table 3: Suds Mileage

| Compositions | SC3 Hydrophobin Concentration in Composition [ppm] | H1 [mm] | H2 [mm] | % suds height drop H2 vs H1 |
|---|---|---|---|---|
| Comparative Composition D | 12 | 11 | 10 | 9% |
| Comparative Composition E | 0 | 11 | 10 | 9% |

The results confirm that Comparative Composition D detergent solution comprising *Schizophyllum commune* hydrophobin SC3 (SEQ ID NO: 13), a class I hydrophobin outside the scope of the present invention, does not have a superior suds profile when compared to Comparative Composition E detergent solution without the class I hydrophobin protein outside the scope of the present invention, both in view of absolute suds height build-up as in view of sustaining the suds height in presence of greasy soil (*e.g.,* olive oil).

Example 4 - Exemplary Manual Dish-Washing Detergent Composition

Table 4 exemplifies a manual dish-washing detergent composition comprising *Neurospora crassa* EAS Hydrophobin (SEQ ID NO: 9) or its variants His-Ubi-EAS (SEQ ID NO: 11) according to the invention.

Table 4: Detergent Composition

| Ingredient | Wt% |
|---|---|
| Sodium alkyl ethoxy sulfate (C1213EO0.6S) | 22.91% |
| n-C12-14 Di Methyl Amine Oxide | 7.64% |
| Lutensol XP80 (non-ionic surfactant supplied by BASF) | 0.45% |
| Sodium Chloride | 1.2% |
| Poly Propylene Glycol (MW 2000) | 1% |
| Ethanol | 2% |
| Sodium Hydroxide | 0.24% |
| *Neurospora crassa* EAS Hydrophobin (SEQ ID NO: 9) or *Neurospora crassa* His-Ubi-EAS Hydrophobin (SEQ ID NO: 11) | 0.5% |
| Minors (perfume, preservative, dye) + water | To 100 % |
| pH (@ 10% solution) | 9 |

All percentages and ratios given for proteins are based on active protein. All percentages and ratios herein are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A cleaning composition comprising:

    a) from 1 wt% to 60 wt%, preferably from 5 wt% to 50 wt%, by weight of the cleaning composition of a surfactant system comprising one or more anionic surfactants and one or more co-surfactants selected from the group consisting of amphoteric surfactant, zwitterionic surfactant, and mixtures thereof; and
    b) from 0.001 wt% to 5 wt%, preferably from 0.1 wt% to 1 wt%, by weight of the cleaning composition, based on active protein, of one or more subclass EAS hydrophobins having at least 80%, preferably at least 85%, preferably at least 90%, preferably at least 95%, preferably at least 98% or even 100% amino acid identity as calculated over the entire length of the sequence aligned against the entire length of at least one or more reference sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 11, preferably SEQ ID NO: 9;

    preferably wherein the cleaning composition is a liquid manual dishwashing cleaning composition.

2. The composition according to claim 1, further comprising one or more carbohydrates selected from the group comprising O-glycan, N-glycan, and mixtures thereof.

3. The composition according to any preceding claims, wherein the weight ratio of the anionic surfactants to the co-surfactants is less than 9:1, more preferably from 5:1 to 1:1, more preferably from 4:1 to 2:1.

4. The composition according to any preceding claims, wherein the amphoteric surfactant is amine oxide surfactant and the zwitterionic surfactant is betaine surfactant.

5. The composition according to any preceding claims, wherein the anionic surfactants are selected from the group consisting of: alkyl sulfates, alkyl alkoxy sulfates, alkyl benzene sulfonates, paraffin sulfonates, and mixtures thereof, preferably a mixture of alkyl sulfates and alkyl ethoxy sulfates.

6. The composition according to any preceding claims, wherein the anionic surfactants are a mixture of alkyl sulfates and alkyl alkoxy sulfates, wherein the co-surfactants are alkyl dimethyl amine oxides, and wherein the weight ratio of the anionic surfactants to the co-surfactants is from 4:1 to 2:1.

7. The composition according to claim 6 wherein the anionic surfactant is a mixture of alkyl sulfate and alkyl ethoxy sulfate, wherein the mixture has a combined mol average ethoxylation degree of less than 5, preferably less than 3, more preferably less than 2 and more than 0.5 and preferably an average level of branching of from 5% to 40%, more preferably from 10% to 35%, and even more preferably from 20% to 30%.

8. The composition according to any preceding claims, further comprising a chelant, preferably selected from the group comprising carboxylate chelants, amino carboxylate chelants, amino phosphonate chelants, and mixtures thereof, preferably selected from the group of MGDA (methylglycine-N,N-diacetic acid), GLDA (glutamic-N,N- diacetic acid), and mixtures thereof.

9. The composition according to any preceding claims, further comprising one or more enzymes selected from the group consisting of amylases, lipases, proteases, cellulase, lipoxygenases, diol synthases, and mixtures thereof.

10. A method of contacting a cleaning composition according to any preceding claims with a surface.

11. A method of manually washing soiled items, preferably dishware, comprising contacting a cleaning composition according to claims 1 to 9 with a surface, preferably dishware, wherein said composition modifies the hydrophobicity of said surface as a result of said contacting step.

12. A method of improving suds longevity in a washing process for washing soiled articles, preferably dishware, comprising the steps of:

    a) delivering a cleaning composition according to claims 1 to 9 to a volume of water to form a wash liquor; and
    b) immersing the soiled articles into said wash liquor.

**13.** The method according to claim 12, wherein the subclass EAS hydrophobins are present at a concentration of from 0.005 ppm to 60 ppm, preferably from 0.02 ppm to 12 ppm, based on active protein, in an aqueous wash liquor during said washing process.

**14.** Use of one or more subclass EAS hydrophobins to improve suds longevity in an aqueous wash liquor during a washing process, wherein the subclass EAS hydrophobins have at least 80%, preferably at least 85%, preferably at least 90%, preferably at least 95%, preferably at least 98% or even 100% amino acid identity as calculated over the entire length of the sequence aligned against the entire length of at least one or more reference sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 11, preferably SEQ ID NO: 9.

**15.** Use according to claim 14 wherein the aqueous wash liquor further comprises a surfactant system comprising one or more anionic surfactants and one or more co-surfactants selected from the group consisting of amphoteric surfactant, zwitterionic surfactant, and mixtures thereof.

Figure 1

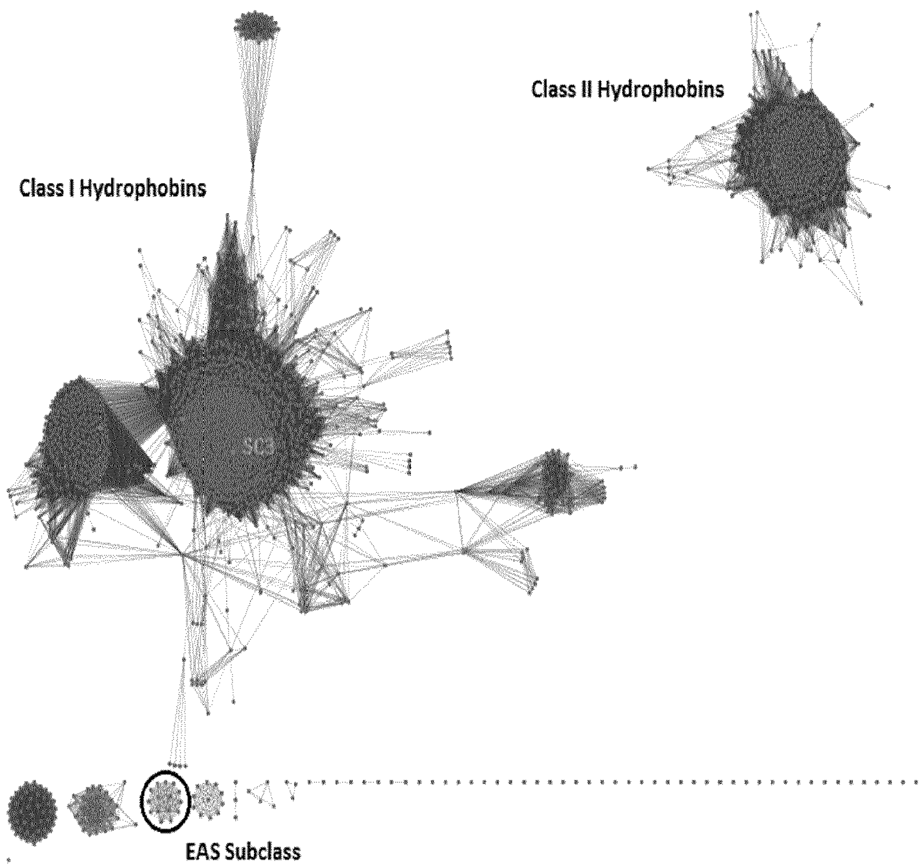
Class II Hydrophobins
Class I Hydrophobins
SC3
EAS Subclass

Europäisches Patentamt
European Patent Office
Office européen des brevets

# EUROPEAN SEARCH REPORT

Application Number
EP 18 17 8698

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2012/137147 A1 (DANISCO US INC [US]; SHIPOVSKOV STEPAN [DK]; JENSEN LENE BOJSEN [DK];) 11 October 2012 (2012-10-11)<br>* page 1, paragraph 1 - page 2, paragraph 2 *<br>* page 3, paragraph 1 - page 4, paragraph 1; figures 1-5, 13; example 1; sequences 6,8 *<br>* page 7, last paragraph - page 8, paragraph 2 *<br>* page 21, lines 13-21 *<br>* page 59, paragraph 2 *<br>----- | 1-15 | INV.<br>C11D3/38<br>C07K14/37<br>C11D1/94<br>C11D11/00 |
| T | Anonymous: "Laundrypedia -Ingredients ¦ Innovation ¦ Ariel",<br>Laundrypedia,<br>19 April 2018 (2018-04-19), pages 1-3, XP055468874,<br>Retrieved from the Internet:<br>URL:https://www.ariel.co.uk/en-gb/about-ariel/ingredients/laundrypedia-ingredients<br>[retrieved on 2018-04-19]<br>* page 2 *<br>----- | | TECHNICAL FIELDS SEARCHED (IPC)<br>C11D<br>C07K |
| A,D | US 2009/101167 A1 (BOECKH DIETER [DE] ET AL) 23 April 2009 (2009-04-23)<br>* paragraphs [0001] - [0006], [0013] - [0015], [0019], [0025] - [0028], [0042], [0091] - [0104], [0112], [0129] - [0131], [0135]; examples 4, 11-15; sequence 6 *<br>----- | 1-15 | |

The present search report has been drawn up for all claims

1

| Place of search | Date of completion of the search | Examiner |
|---|---|---|
| The Hague | 19 October 2018 | Marttin, Emmeline |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 18 17 8698

This annex lists the patent family members relating to the patent documents cited in the above-mentioned European search report. The members are as contained in the European Patent Office EDP file on The European Patent Office is in no way liable for these particulars which are merely given for the purpose of information.

19-10-2018

| Patent document cited in search report | Publication date | Patent family member(s) | Publication date |
|---|---|---|---|
| WO 2012137147 A1 | 11-10-2012 | AR 085845 A1 | 30-10-2013 |
| | | AU 2012241055 A1 | 15-08-2013 |
| | | BR 112013025811 A2 | 29-11-2016 |
| | | CA 2830579 A1 | 11-10-2012 |
| | | EP 2694537 A1 | 12-02-2014 |
| | | JP 6027092 B2 | 16-11-2016 |
| | | JP 2014516509 A | 17-07-2014 |
| | | KR 20140024365 A | 28-02-2014 |
| | | RU 2013149861 A | 20-05-2015 |
| | | US 2014031272 A1 | 30-01-2014 |
| | | WO 2012137147 A1 | 11-10-2012 |
| US 2009101167 A1 | 23-04-2009 | AT 485359 T | 15-11-2010 |
| | | AU 2006274836 A1 | 08-02-2007 |
| | | BR PI0614703 A2 | 23-08-2011 |
| | | CA 2617092 A1 | 08-02-2007 |
| | | CN 101233220 A | 30-07-2008 |
| | | EP 1913123 A1 | 23-04-2008 |
| | | ES 2352970 T3 | 24-02-2011 |
| | | JP 5105441 B2 | 26-12-2012 |
| | | JP 2009503280 A | 29-01-2009 |
| | | KR 20080041228 A | 09-05-2008 |
| | | US 2009101167 A1 | 23-04-2009 |
| | | WO 2007014897 A1 | 08-02-2007 |
| | | ZA 200801881 B | 26-08-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090101167 A
- US 20140031272 A
- WO 201219844 A
- WO 201219849 A
- WO 201219848 A
- US 3915903 A
- WO 2007135645 A


### Non-patent literature cited in the description


- **COOPER, A. et al.** *Colloids Surf., A: Physiochemical and Engineering Aspects,* 2017
- **SCHOR, M. et al.** *Trends Biochem. Sci.,* 2016, vol. 41 (7), 610-620
- **WOSTEN, H. A. B.** *Annu. Rev. Microbiol.,* 2001, vol. 55, 625-646
- **KYTE ; DOOLITTLE.** *J. Mol. Biol.,* 1982, vol. 157, 105-132
- **WINEFIELD, R. D. et al.** *Fungal Genet. Biol.,* 2007, vol. 44 (4), 250-257
- **PETERSEN TN. ; BRUNAK S. ; HEIJNE G. ; NIELSEN H.** *Nature Methods,* 2011, vol. 8, 785-786
- **NEEDLEMAN ; WUNSCH.** *J. Mol. Biol.,* 1970, vol. 48, 443-453
- **HENIKOFF S. ; HENIKOFF J.G.** *P.N.A.S. USA,* 1992, vol. 89, 10915-10919
- **ROBERT LAUGHLIN.** The Aqueous Phase Behaviour of Surfactants. Academic Press, 1994, 538-542

SEQUENCE LISTING

<110> The Procter & Gamble Company

<120> Cleaning composition

<130> CM04909FM

<160> 13

<170> PatentIn version 3.5

<210> 1
<211> 108
<212> PRT
<213> Neurospora crassa

<400> 1

```
Met Gln Phe Thr Ser Val Phe Thr Ile Leu Ala Ile Ala Met Thr Ala
1               5                   10                  15

Ala Ala Ala Pro Ala Glu Val Val Pro Arg Ala Thr Thr Ile Gly Pro
            20                  25                  30

Asn Thr Cys Ser Ile Asp Asp Tyr Lys Pro Tyr Cys Cys Gln Ser Met
        35                  40                  45

Ser Gly Pro Ala Gly Ser Pro Gly Leu Leu Asn Leu Ile Pro Val Asp
    50                  55                  60

Leu Ser Ala Ser Leu Gly Cys Val Val Gly Val Ile Gly Ser Gln Cys
65                  70                  75                  80

Gly Ala Ser Val Lys Cys Cys Lys Asp Asp Val Thr Asn Thr Gly Asn
                85                  90                  95

Ser Phe Leu Ile Ile Asn Ala Ala Asn Cys Val Ala
            100                 105
```

<210> 2
<211> 91
<212> PRT
<213> Neurospora tetrasperma

<400> 2

```
Leu Ala Ile Ala Met Thr Ala Ala Ala Ala Pro Ala Glu Val Val Pro
1               5                   10                  15

Arg Ala Thr Thr Ile Gly Pro Asn Thr Cys Ser Ile Asp Asp Tyr Lys
            20                  25                  30

Pro Tyr Cys Cys Gln Ser Met Ser Gly Pro Ala Gly Ser Pro Gly Leu
```

```
        35                  40                  45

Leu Asn Leu Ile Pro Val Asp Leu Ser Ala Ser Leu Gly Cys Val Val
    50                  55                  60

Gly Val Ile Gly Ser Gln Cys Gly Ala Ser Val Lys Cys Cys Lys Asp
65                  70                  75                  80

Asp Val Thr Asn Thr Gly Asn Ser Phe Leu Ile
                85                  90


<210>  3
<211>  91
<212>  PRT
<213>  Neurospora discreta

<400>  3

Leu Ala Val Ala Met Thr Ala Ala Ala Ala Pro Ala Glu Val Val Pro
1               5                   10                  15

Arg Ala Gln Thr Ile Gly Pro Asn Thr Cys Ser Ile Asp Asp Tyr Lys
            20                  25                  30

Pro Tyr Cys Cys Gln Ser Met Ser Gly Pro Ala Gly Ser Pro Gly Leu
        35                  40                  45

Leu Asn Leu Ile Pro Val Asp Leu Ser Ala Ser Leu Gly Cys Val Val
    50                  55                  60

Gly Val Ile Gly Ser Gln Cys Gly Ala Ser Val Lys Cys Cys Lys Asp
65                  70                  75                  80

Asp Val Thr Asn Thr Gly Asn Ser Phe Leu Ile
                85                  90


<210>  4
<211>  91
<212>  PRT
<213>  Neurospora tetrasperma

<400>  4

Leu Ala Ile Ala Met Thr Ala Ala Ala Ala Pro Ala Glu Val Val Pro
1               5                   10                  15

Arg Ala Thr Thr Ile Gly Pro Asn Thr Cys Ser Ile Asp Asp Tyr Lys
            20                  25                  30

Pro Tyr Cys Cys Gln Ser Met Ser Gly Pro Ala Gly Ser Pro Gly Leu
        35                  40                  45
```

```
Leu Asn Leu Ile Pro Val Asp Leu Ser Ala Ser Leu Gly Cys Val Val
    50                  55                  60

Gly Val Ile Gly Ser Gln Cys Gly Ala Ser Val Lys Cys Cys Lys Asp
65                  70                  75                  80

Gly Val Thr Asn Thr Gly Asn Ser Phe Leu Ile
                85                  90


<210>  5
<211>  91
<212>  PRT
<213>  Neurospora sitophila

<400>  5

Leu Ala Ile Ala Met Thr Ala Ala Ala Ala Pro Ala Glu Val Val Pro
1               5                   10                  15

Arg Ala Thr Thr Ile Asp Ala Asn Thr Cys Ser Ile Asp Asp Tyr Lys
            20                  25                  30

Pro Tyr Cys Cys Gln Ser Met Ser Gly Pro Ala Gly Ser Pro Gly Leu
        35                  40                  45

Leu Asn Leu Ile Pro Val Asp Leu Ser Ala Ser Leu Gly Cys Val Val
    50                  55                  60

Gly Val Ile Gly Ser Gln Cys Gly Ala Ser Val Lys Cys Cys Lys Asp
65                  70                  75                  80

Asp Val Thr Asn Thr Gly Asn Ser Phe Leu Ile
                85                  90


<210>  6
<211>  88
<212>  PRT
<213>  Neurospora terricola

<400>  6

Leu Thr Ile Ala Met Thr Ala Ala Ala Ala Pro Ala Glu Val Val Ala
1               5                   10                  15

Arg Thr Thr Ile Thr Pro Glu Ser Cys Thr Ile Pro Asn Tyr Lys Pro
            20                  25                  30

Tyr Cys Cys Gln Ser Leu Ser Gly Thr Gly Gly Gly Leu Leu Gly Leu
        35                  40                  45
```

Leu Pro Ile Asp Leu Ser Ala Thr Leu Gly Cys Val Val Gly Val Ile
50 55 60

Gly Gly Gln Cys Ser Ala Ser Val Lys Cys Cys Lys Ser Asp Val Thr
65 70 75 80

Asn Thr Gly Asn Ser Leu Ile Ile
85

<210> 7
<211> 89
<212> PRT
<213> Neurospora lineolata

<400> 7

Leu Thr Ile Ala Met Thr Ala Ala Ala Ala Pro Ala Glu Val Val Ala
1 5 10 15

Arg Thr Thr Ile Thr Pro Glu Ser Cys Ser Ile Pro Asn Tyr Lys Pro
20 25 30

Tyr Cys Cys Gln Ser Leu Ser Ser Thr Gly Gly Gly Ile Leu Gly Ala
35 40 45

Leu Leu Gly Val Asp Leu Ser Ala Thr Leu Gly Cys Val Val Gly Ala
50 55 60

Val Gly Gly Gln Cys Ser Ala Ser Val Lys Cys Cys Lys Ser Asp Ala
65 70 75 80

Thr Asn Thr Gly Asn Ser Leu Ile Leu
85

<210> 8
<211> 108
<212> PRT
<213> Sordaria macrospora

<400> 8

Met Gln Phe Ser Ser Ile Ala Thr Phe Leu Thr Leu Ala Met Thr Ala
1 5 10 15

Ala Ala Ala Pro Ala Glu Leu Val Ser Arg Thr Thr Pro Val Thr Pro
20 25 30

Ser Thr Cys Ala Gly Asp Tyr Lys Pro Tyr Cys Cys Asn Ser Glu Pro
35 40 45

Pro Thr Gly Leu Leu Pro Gln Val Ile Ala Gly Leu Leu Gly Val Asn
    50                  55                  60

Val Asn Ala Leu Leu Ser Cys Thr Val Gly Val Ile Gly Gly Gln Cys
65                  70                  75                  80

Ser Asn Asn Val Lys Cys Cys Asn Gly Asn Ala Val Asn Asn Gly Asn
                85                  90                  95

Ser Leu Ile Phe Ile Gly Asn Val Gln Cys Leu Leu
            100                 105

<210>  9
<211>  82
<212>  PRT
<213>  Neurospora crassa

<400>  9

Ala Thr Thr Ile Gly Pro Asn Thr Cys Ser Ile Asp Asp Tyr Lys Pro
1               5                   10                  15

Tyr Cys Cys Gln Ser Met Ser Gly Pro Ala Gly Ser Pro Gly Leu Leu
            20                  25                  30

Asn Leu Ile Pro Val Asp Leu Ser Ala Ser Leu Gly Cys Val Val Gly
        35                  40                  45

Val Ile Gly Ser Gln Cys Gly Ala Ser Val Lys Cys Cys Lys Asp Asp
    50                  55                  60

Val Thr Asn Thr Gly Asn Ser Phe Leu Ile Ile Asn Ala Ala Asn Cys
65                  70                  75                  80

Val Ala

<210>  10
<211>  516
<212>  DNA
<213>  None

<400>  10
atgcaccacc accaccatca catgcaaatc ttcgttaaaa ccctgaccgg caaaaccatc      60

accctggaag ttgaaccgag cgacaccatc gagaacgtga aggcgaaaat ccaggacaag     120

gaaggcattc cgccggatca gcaacgtctg atcttcgcgg gcaaacagct ggaggacggt     180

cgtaccctga gcgattacaa cattcaaaag gaaagcaccc tgcacctggt tctgcgtctg     240

cgtggtggcg agaacctgta ttttcagggc gcgaccacca tcggtccgaa cacctgcagc     300

attgacgatt acaaaccgta ttgctgccaa agcatgagcg gtccggcggg cagcccgggt 360

ctgctgaacc tgattccggt ggatctgagc gcgagcctgg gttgcgtggt tggcgtgatt 420

ggtagccaat gcggtgcgag cgttaagtgc tgcaaagacg atgttaccaa taccggcaat 480

agcttcctga ttatcaatgc ggcgaactgc gtggcg 516

<210> 11
<211> 172
<212> PRT
<213> Neurospora crassa

<400> 11

Met His His His His His His Met Gln Ile Phe Val Lys Thr Leu Thr
1               5                   10                  15

Gly Lys Thr Ile Thr Leu Glu Val Glu Pro Ser Asp Thr Ile Glu Asn
            20                  25                  30

Val Lys Ala Lys Ile Gln Asp Lys Glu Gly Ile Pro Pro Asp Gln Gln
        35                  40                  45

Arg Leu Ile Phe Ala Gly Lys Gln Leu Glu Asp Gly Arg Thr Leu Ser
    50                  55                  60

Asp Tyr Asn Ile Gln Lys Glu Ser Thr Leu His Leu Val Leu Arg Leu
65                  70                  75                  80

Arg Gly Gly Glu Asn Leu Tyr Phe Gln Gly Ala Thr Thr Ile Gly Pro
                85                  90                  95

Asn Thr Cys Ser Ile Asp Asp Tyr Lys Pro Tyr Cys Cys Gln Ser Met
            100                 105                 110

Ser Gly Pro Ala Gly Ser Pro Gly Leu Leu Asn Leu Ile Pro Val Asp
        115                 120                 125

Leu Ser Ala Ser Leu Gly Cys Val Val Gly Val Ile Gly Ser Gln Cys
    130                 135                 140

Gly Ala Ser Val Lys Cys Cys Lys Asp Asp Val Thr Asn Thr Gly Asn
145                 150                 155                 160

Ser Phe Leu Ile Ile Asn Ala Ala Asn Cys Val Ala
                165                 170

<210> 12
<211> 71
<212> PRT

<213> Trichoderma reesei

<400> 12

```
Ala Val Cys Pro Thr Gly Leu Phe Ser Asn Pro Leu Cys Cys Ala Thr
1               5                   10                  15

Asn Val Leu Asp Leu Ile Gly Val Asp Cys Lys Thr Pro Thr Ile Ala
            20                  25                  30

Val Asp Thr Gly Ala Ile Phe Gln Ala His Cys Ala Ser Lys Gly Ser
        35                  40                  45

Lys Pro Leu Cys Cys Val Ala Pro Val Ala Asp Gln Ala Leu Leu Cys
    50                  55                  60

Gln Lys Ala Ile Gly Thr Phe
65                  70
```

<210> 13
<211> 112
<212> PRT
<213> Schizophyllum commune

<400> 13

```
Gly Gly His Pro Gly Thr Thr Thr Pro Pro Val Thr Thr Thr Val Thr
1               5                   10                  15

Val Thr Thr Pro Pro Ser Thr Thr Thr Ile Ala Ala Gly Gly Thr Cys
            20                  25                  30

Thr Thr Gly Ser Leu Ser Cys Cys Asn Gln Val Gln Ser Ala Ser Ser
        35                  40                  45

Ser Pro Val Thr Ala Leu Leu Gly Leu Leu Gly Ile Val Leu Ser Asp
    50                  55                  60

Leu Asn Val Leu Val Gly Ile Ser Cys Ser Pro Leu Thr Val Ile Gly
65                  70                  75                  80

Val Gly Gly Ser Gly Cys Ser Ala Gln Thr Val Cys Cys Glu Asn Thr
                85                  90                  95

Gln Phe Asn Gly Leu Ile Asn Ile Gly Cys Thr Pro Ile Asn Ile Leu
            100                 105                 110
```